# EUROPEAN PATENT APPLICATION

(11) **EP 4 169 936 A1**
(43) Date of publication of application: **26.04.2023**
(21) Application number: 21382952.6
(22) Date of filing: 22.10.2021
(51) Int. Cl.: C07K 14/52, C07K 16/28, A01K 67/027

(54) **PATIENT-DERIVED FULLY-HUMANIZED MOUSE AND METHOD FOR PREDICTING THE RESPONSE TO MULTIPLE MYELOMA TREATMENTS USING THE SAME**

(71) Applicant: FUNDACIÓN PARA LA INVESTIGACIÓN BIOMÉDICA DEL HOSPITAL 12 DE OCTUBRE, 28041 Madrid (ES); Centro de Investigaciones Energéticas, Medioambientales y Tecnológicas O.A., M.P. (CIEMAT), 28040 Madrid (ES)
(72) Inventor: Valeri Lozano, Antonio, 28041 Madrid (ES); Castellano Esparza, Eva, 28041 Madrid (ES); García Ortiz, Almudena, 28041 Madrid (ES); Maroto Martín, Elena, 28041 Madrid (ES); Encinas Mayoral, Jessica, 28041 Madrid (ES); Alonso Fernández, Rafael, 28041 Madrid (ES); Río Galdo, Paula, 28040 Madrid (ES); Martínez López, Joaquín, 28041 Madrid (ES)
(74) Representative: Manuel Illescas y Asociados, S.L.

(57) **Abstract**

Human immune system transgenic mouse having the genotype Rag2^{-/-}, II2rg^{-/-}, Sirpa^{NOD}, Flt3^{-/-}, engrafted with bone marrow mononuclear cells (BMMCs) obtained from a subject having multiple myeloma, administered with antibodies recognizing human gp130, and administered with human FLT3 ligand (hFLT3-L). Method for predicting the anti-tumour response of the subject to a multiple myeloma treatment, comprising evaluating the anti-tumour response of the humanized mouse of the invention to the multiple myeloma treatment.

## Description

### TECHNICAL FIELD

The present invention relates to a humanized mouse useful as an animal model for multiple myeloma. The animal model allows determining the efficacy of therapies against multiple myeloma, in particular, adoptive cell transfer immunotherapies.

### BACKGROUND ART

Mouse *in vivo* models are essential to assay the efficacy and safety of new therapies against human diseases, as hematologic malignancies. Although different approaches have been proposed for multiple myeloma (MM) *in vivo* models, most of them are characterized by low clinical predictability because of their inability to reproduce some MM features and patients' bone marrow microenvironment.

The first *in vivo* models for the study of MM disease were based on the spontaneous generation of age-associated malignancies in some mouse strains. C57BL/KalwRij mice strains generate B cell lymphoproliferative disorders as Waldenstrom's disease or MM. Plasma cells from these mice are transplanted into syngeneic mice to reproduce the pathology, generating models like 5TMM and other variants based on derived cell lines (5T2 and 5T33) (Libouban, 2015). However, these models present relevant disadvantages such as deficient clonal diversity together with the development of a murine disease that differs from human pathology. Moreover, the murine background of this model impedes the study of human-specific targets added to the lack of human bone marrow microenvironment.

Later, the NOD Severe Combined Immunodeficiency (SCID) gamma (NSG) mouse, a xenograft model of MM, was designed. MM human cell lines (U266B1, RPMI 8226, JJN3, OPM 2) were intravenously infused in NSG mice with successful engraftment rates (Rossi et al., 2018). Although lytic lesions have been described, this model inaccurately reflects all the disease features, and lack of competent immune system and, again, bone marrow microenvironment. In addition, this model does not mirror the slow progress of MM disease because most of the commonly used cell lines are derived from plasma cell leukemia inducing an aggressive disease with short-term latency.

Another model of the prior art, the humanized model SCID-hu, is a heterotopic model based on an implanted fetal human bone chip (FBC) in irradiated immune-deficient mice (Yaccoby et al., 1998). In this system, MM cell lines or primary cells home exclusively to FBC instead of murine bone. Nevertheless, this model presents relevant drawbacks as sample availability, ethical considerations, and the fact that fetal bone does not resemble the MM adult microenvironment. SCID-synth-hu, a model derived from SCID-hu, replaces fetal bone for synthetic bone scaffolds (Calimeri et al., 2011). However, this model does not resolve the absence of a human bone marrow niche and requires a sophisticated manufacturing process.

In recent years, patient-derived xenograft (PDX) models have also been developed. PDX models are immunodeficient mice engrafted with patients' tumor cells or tissues. However, one of the main obstacles in MM models is that primary human MM cells are not able to engraft bone marrow from conventional immunodeficient mouse strains, mainly because of the lack of cell-cell interactions and interspecies cross-reactivity of certain cytokines essential to sustain human plasma cells. This is the case of IL-6, because murine IL-6 acts only on murine cells, while human IL-6 stimulates human and murine cells. In fact, the continuous exogenous administration of human IL-6, which could improve MM cells engraftment, due to human IL-6 recognition by IL-6 murine receptor, induces mice cachexia. IL-6R forms complexes with gp130 to trigger downstream signaling. The use of anti-gp130 monoclonal antibodies in a SCID model engrafted with patient-derived MM cells initiates the IL-6R signaling pathway promoting tumor growth without any obvious toxicity or mortality (Rebouissou et al., 1998), which reflects the high specie recognition of these anti-gp130 antibodies.

MIS(^{KI})TRG6, a combined PDX-Human Immune System (HIS) model, is a humanized mouse strain that contains knock-in alleles that express human genes relevant for innate immune cell development and hematopoiesis (M-CSF, IL-3, GM-CSF, thrombopoietin, and SIRPα) and MM cells proliferation (human IL-6) (Das et al., 2016). The strength of this model is the feasibility of patient-derived non-malignant cells' growth along with tumor cells, whose interaction contributes to MM survival. However, MISTRG mice develop severe anemia resulting in a short lifespan and, consequently, strain production has been discontinued.

In the HIS model BRGSF, BALB/c mice have a genotype Rag2^{-/-} Il2rg^{-/-} Sirpa^{NOD}Flk2/Flt3^{-/-}that neither presents murine T, B, or Natural Killer (NK) cells, nor expresses murine FLT3 receptor. The latter prevents murine myeloid cells differentiation and eliminates FLT3-L competition of human myeloid cells promoting the presence of conventional (cDC) and plasmacytoid dendritic cells (pDC) that, along with macrophages differentiated from CD34⁺ progenitor cells, enable antigen presentation function. Consequently, the T cell population is potentiated as NK cells are, whose homeostasis and function are regulated by the interaction with other immune cells, mainly macrophages, dendritic cells (DC), and T cells. In this model, Treg and myeloid-derived suppressor cells (MDSC) have also been detected. That is why this model not only generates a human immune system that could respond against the tested immunotherapy, but also resembles the immunosuppressive microenvironment present in MM bone marrow, which may also have an impact on its anti-tumor efficacy. Furthermore, BRGSF strain does not raise any problem in lifespan, at least when human CD34⁺ hematopoietic stem cells are injected (Lopez-Lastra et al., 2017). Nevertheless, additional strategies must be incorporated into this model in order to obtain an animal model able to properly sustain MM plasma cells.

As a summary, the animal models of the prior art present one or more of the following disadvantages: deficient clonal diversity; development of a murine disease that differs from human pathology; the murine background impedes the study of human-specific targets; lack of human bone marrow microenvironment; ethical considerations related to the use of fetal bones; fetal bones does not resemble MM adult microenvironment; requirement of a sophisticated manufacturing process; short lifespan of the model; and models are not able to properly sustain MM plasma cells.

Accordingly, there is a need in the art for a patient-derived xenograft (PDX) MM mouse model with human immune system (HIS) reconstitution which overcomes one or more of the disadvantages of the animal models of the prior art; provides patient-derived plasma cells maintenance and provides healthy immune system reconstitution.

### SUMMARY OF INVENTION

As used herein, "human immune system mouse" (HIS mouse) refers to a humanized mouse model comprising a human immune system, used to study and modulate the interactions between immune components and tumors of human origin.

As used herein, "Rag2" refers to recombination activating gene 2, a lymphocyte-specific gene which expresses the protein RAG2. Together with RAG1 protein, RAG2 forms a recombinase, a protein complex required for the process of recombination in which the variable regions of immunoglobulin and T cell receptor genes are assembled in order to develop B and T lymphocytes. RAG2 is essential for the generation of mature B and T lymphocytes.

As used herein, "Il2rg" refers to interleukin-2 receptor gamma chain gene (also known as "common gamma chain (yc)" and "CD132"), which expresses the protein IL2RG. IL2RG is a cytokine receptor subunit that is common to the receptor complexes for at least six different interleukin receptors: IL-2, IL-4, IL-7, IL-9, IL-15, and IL-21 receptor. IL2RG is a member of the type I cytokine receptor family expressed on most lymphocyte populations, including NK cells.

As uses herein, "Sirpa" refers to signal regulatory protein α gene (also known as "Sirpα" and "CD172a"), which expresses the protein signal regulatory protein α (SIRPα). SIRPα is a regulatory membrane glycoprotein expressed mainly by myeloid cells and also by stem cells or neurons. SIRPα acts as an inhibitory receptor and interacts with the broadly expressed transmembrane protein CD47, also called the "don't eat me" signal. This interaction negatively controls the effector function of innate immune cells such as host cell phagocytosis.

As used herein, "Sirpa^{NOD}" refers to the polymorphic Sirpa gene from the homozygous non-obese diabetic (NOD) mouse.

As used herein, "CD34" refers to a transmembrane phosphoglycoprotein protein. CD34 is expressed on hematopoietic stem cells as a cell surface glycoprotein and functions as a cell-cell adhesion factor. It may also mediate the attachment of hematopoietic stem cells to the bone marrow extracellular matrix or directly to stromal cells.

As used herein, "Flt3" refers to FMS-related tyrosine kinase 3 gene (also known as "fetal liver kinase-2 (Flk2)" and "CD135"), which expresses the protein FLT3. FLT3 is a cytokine receptor which belongs to the receptor tyrosine kinase class III. FLT3 is the receptor for the cytokine FLT3 ligand (FLT3-L). FLT3 is expressed on the surface of many hematopoietic progenitor cells. Signaling of FLT3 is important for the normal development of hematopoietic stem cells and progenitor cells and for the differentiation of the myeloid lineage.

As used herein, "hFLT3-L" refers to human FLT3 ligand.

As used herein, "bone marrow mononuclear cells (BMMCs)" refers to any bone marrow cell having a round nucleus. These cells consist of lymphocytes (T cells, B cells, NK cells) and monocytes, whereas erythrocytes and platelets have no nuclei, and granulocytes (neutrophils, basophils, and eosinophils) have multi-lobed nuclei.

As used herein, "gp130" refers to glycoprotein 130, a transmembrane protein which forms one subunit of the type I cytokine receptor within the IL-6 receptor family.

As used herein, "BRGSF mouse" refers to a BALB/c mouse with a genotype Rag2^{-/-} Il2rg^{-/-}Sirpa^{NOD} Flk2/Flt3^{-/-}. BRGSF mouse neither presents murine T, B, or NK cells, nor expresses murine FLT3 receptor.

As used herein, "chimeric antigen receptor (CAR)" refers to a synthetic receptor consisting of an extracellular antigen-binding domain that recognizes a tumor-associated antigen (TAA) and an intracellular signaling domain derived from the T cell receptor (TCR), most commonly CD3ζ (the zeta chain associated with the T cell receptor complex). CAR may also include one or two co-stimulatory domains, usually derived from CD28 and/or 4-1BB.

As used herein, "adoptive cell therapy" (also known as "adoptive cell transfer" and "cellular adoptive immunotherapy") refers to a type of immunotherapy in which immune cells (such as Natural Killer (NK) cells, T cells, macrophages, Natural Killer T (NKT) cells, and gamma delta T (γδ T) cells) are given to a patient to help the body fight diseases, such as cancer. In cancer therapy, immune cells are usually taken from the patient's own blood, bone marrow or tumor tissue, grown in large numbers in the laboratory, and then given back to the patient to help the immune system fight the cancer. Sometimes, the immune cells are modified in the laboratory to make them better able to target the patient's cancer cells and kill them. Types of adoptive cell therapy include CAR-NK cell therapy, CAR T-cell therapy, CAR-macrophage cell therapy, CAR-NKT cell therapy, and CAR-γδ T cell therapy.

As used herein, "Natural Killer T cells (NKT)" refers to a group of T cells that share properties of both T cells and NK cells. Many of these cells recognize the CD1d molecule, an antigen-presenting molecule that binds lipids and glycolipids.

As used herein, "gamma delta T cells (γδ T cells)" refers to T cells that have a distinctive T cell receptor (TCR) on their surface. Most T cells are αβ (alpha beta) T cells with TCR composed of two glycoprotein chains called α (alpha) and β (beta) TCR chains. In contrast, gamma delta (γδ) T cells have a TCR that is made up of one γ (gamma) chain and one δ (delta) chain.

The term "BCMA" refers to B-cell maturation antigen, also known as tumor necrosis factor receptor superfamily member 17 (TNFRSF17). BCMA is a member of the TNF-receptor superfamily. BCMA is preferentially expressed in mature B lymphocytes and is important for B cell development and autoimmune response.

The technical problem to be solved is to provide an improved animal model for multiple myeloma providing patient-derived plasma cells maintenance and healthy immune system reconstitution.

The invention as defined in the claims provides a solution to this technical problem.

The present invention provides a human immune system transgenic mouse having the genotype Rag2^{-/-}, Il2rg^{-/-}, Sirpa^{NOD}, Flt3^{-/-},
(a) engrafted with bone marrow mononuclear cells (BMMCs) obtained from a subject having multiple myeloma;
(b) administered with antibodies recognizing human gp130; and
(c) administered with human FLT3 ligand (hFLT3-L).

The mouse of the invention provides several advantages:
- The presence of a near-complete immune system that could respond against the tested therapy, along with a microenvironment that confers resistance to the MM cells by means of the soluble factors and cytokines released by both tumor and healthy cells; as well as the interaction with the main suppressor populations that co-exist in the neoplastic environment which are essential to predict the effectiveness of the therapy in the personalized context of each patient.
- In the animal model, real persistence (and therefore efficacy) of effector cells, i.e. NK cells, which cannot be sustained over time by paracrine signals in current immunodeficiency models existing in the art, can be demonstrated.

- The predictive value of the animal model with regard to toxicity could evaluate the adverse effects of immunotherapies on different hematopoietic lineages, on components of the human stroma, and even on healthy stem cells, corresponding to the same patient.
- Both the engraftment of MM and immune cells obtained from the same MM patient sample, provide an improved mouse model which is able to assess, not only the efficacy of immunotherapies in an almost complete immune context, but also the hematopoietic toxicity.
- The efficacy of an immunotherapy in a patient can be predicted with just one mouse in a sequential model (1 mouse/1 patient/ 1 immunotherapy). This sequential model with just one mouse allows reducing the number of mice necessary to predict the efficacy of an immunotherapy in a patient.

The mouse of the invention is not to be understood as an animal variety.

In embodiments of the mouse of the invention, the mouse has been irradiated prior to engraftment with BMMCs.

In embodiments of the mouse of the invention, the mouse has been irradiated with a dose of 2 Gy.

In embodiments of the mouse of the invention, the BMMCs cells are engrafted intrafemorally.

In embodiments of the mouse of the invention, the administration of the antibodies recognizing human gp130 is repeated every 2 weeks, until week 8 from engraftment.

In embodiments of the mouse of the invention, the antibodies recognizing human gp130 are administered intraperitoneally.

In embodiments of the mouse of the invention, hFLT3-L is administered 3 times per week, for 2 weeks, starting at week 6 from engraftment.

In embodiments of the mouse of the invention, the antibodies recognizing human gp130, are the antibodies B-P8 and B-S12.

In embodiments of the mouse of the invention, hFLT3-L is administered intraperitoneally.

The present invention also provides a method for preparing a human immune system transgenic mouse having the genotype Rag2^{-/-}, Il2rg^{-/-}, Sirpa^{NOD}, Flt3^{-/-}, comprising:
(a) engrafting the mouse with bone marrow mononuclear cells (BMMCs) obtained from a subject having multiple myeloma;
(b) administering antibodies recognizing human gp130 to the mouse; and
(c) administering human FLT3 ligand (hFLT3-L) to the mouse.

The present invention also provides the mouse obtainable by the method for preparing the mouse of the invention.

In embodiments of the method for preparing the mouse of the invention, the mouse has been irradiated prior to engraftment with BMMCs.

In embodiments of the method for preparing the mouse of the invention, the mouse has been irradiated with a dose of 2 Gy.

In embodiments of the method for preparing the mouse of the invention, the BMMCs cells are engrafted intrafemorally.

In embodiments of the method for preparing the mouse of the invention, the administration of the antibodies recognizing human gp130 is repeated every 2 weeks, until week 8 from engraftment.

In embodiments of the method for preparing the mouse of the invention, the antibodies recognizing human gp130 are administered intraperitoneally.

In embodiments of the method for preparing the mouse of the invention, hFLT3-L is administered 3 times per week, for 2 weeks, starting at week 6 from engraftment.

In embodiments of the method for preparing the mouse of the invention, the antibodies recognizing human gp130, are the antibodies B-P8 and B-S12.

In embodiments of the method for preparing the mouse of the invention, hFLT3-L is administered intraperitoneally.

Additionally, the present invention provides a method for predicting the anti-tumour response of a subject to a multiple myeloma treatment, comprising:
(a) preparing a human immune system transgenic mouse of the invention by:
   (i) engrafting the mouse with bone marrow mononuclear cells (BMMCs) obtained from a subject having multiple myeloma;
   (ii) administering antibodies recognizing human gp130 to the mouse; and
   (iii) administering human FLT3 ligand (hFLT3-L) to the mouse;
(b) administering a multiple myeloma treatment to the mouse;
(c) evaluating the anti-tumour response of the mouse to the multiple myeloma treatment; and
(d) predicting the anti-tumour response of the subject to the multiple myeloma treatment based on the evaluation performed in step (c).

The prediction method of the invention evaluates the anti-tumour response to a multiple myeloma treatment in a mouse and predicts the anti-tumour response of a human subject to the multiple myeloma treatment based on said evaluation in the mouse. In view of the above, it is derived that the prediction method of the invention is neither a diagnostic method practiced on the human or animal body nor a method of treatment of the human or animal body by therapy.

In the prediction method of the invention, the anti-tumour response can be evaluated by determining any parameter known in the art. Examples of said parameters include but are not limited to apoptosis, number of cancer cells, and tumour volume. A reduction in the number of cancer cells or in the volume of tumour is indicative of a positive anti-tumour response.

In embodiments of the prediction method of the invention, the multiple myeloma treatment is selected from the group consisting of radiation therapy, gene therapy, and adoptive cell therapy.

In embodiments of the prediction method of the invention, the adoptive cell therapy uses cells selected from the group consisting of Natural Killer (NK) cells, T cells, macrophages, Natural Killer T (NKT) cells, gamma delta T (γδ T) cells, CAR-NK cells, CAR-T cells, CAR-macrophage cells, CAR-NKT cells, and CAR-γδ T cells.

In embodiments of the prediction method of the invention, the multiple myeloma treatment comprises the administration of a chemotherapeutic agent or an antibody.

In embodiments of the prediction method of the invention, the multiple myeloma treatment is administered intravenously.

As used in this specification and the appended claims, the singular forms "a", "an", and "the" include plural referents, unless the content clearly dictates otherwise.

Unless defined otherwise, all the technical and scientific terms have the same meaning as those commonly understood by a person skilled in the art in the field of the invention.

Throughout the description and the claims, the terms "comprises", "comprising" and their variants are not limiting in nature and therefore do not aim to exclude other technical features.

Throughout the description and the claims, the terms "comprise", "comprising" and their variants include, specifically, the term "consisting" or "consisting of".

### BRIEF DESCRIPTION OF DRAWINGS

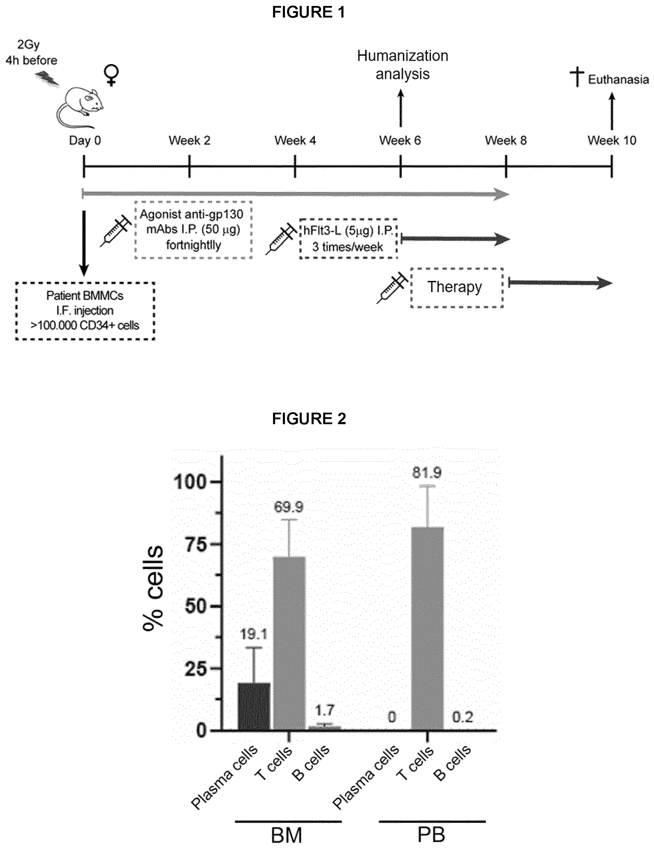

**Figure 1****.** Experimental design for the development of the mouse of the invention, a PDX-HIS murine model of multiple myeloma. Bone marrow mononuclear cells (BMMCs) from MM patient samples are transplanted by intra-femoral injection (I.F.) into previously irradiated BRGSF mice. The antibodies B-P8 and B-S12 are administered intraperitoneally (I.P.) to promote the engraftment and maintenance of the human tumor component in the mouse bone marrow. After 6 weeks, peripheral blood (PB) and bone marrow (BM) samples are extracted from the transplanted animals and the reconstitution of the main healthy human hematological lineages is analyzed. After humanization, hFLT3-L is administered intraperitoneally 3 times per week to promote the development of myeloid populations, along with a treatment with antibodies B-P8 and B-S12. From week 8, the selected therapy is administered intravenously (I.V.). Said therapy may be, for example, autologous or allogeneic adoptive cell therapy. The animals are sacrificed after one or two weeks of treatment (CAR-NK cell therapy, CAR T-cell therapy, CAR-macrophage cell therapy, CAR-NKT cell therapy, and CAR-γδ T cell therapy) and the antitumor efficacy of the therapy is analyzed.
**Figure 2****.** Engraftment of healthy immune populations and pathological plasma cells in the mice at necropsy. Analysis by flow cytometry of the percentage of human (hCD45⁺) plasma cells, T lymphocytes, and B lymphocytes, in the bone marrow (BM) of the transplanted femur and peripheral blood (PB). Mean values (indicated with numbers on the histograms) ± SEM are represented (n = 6).

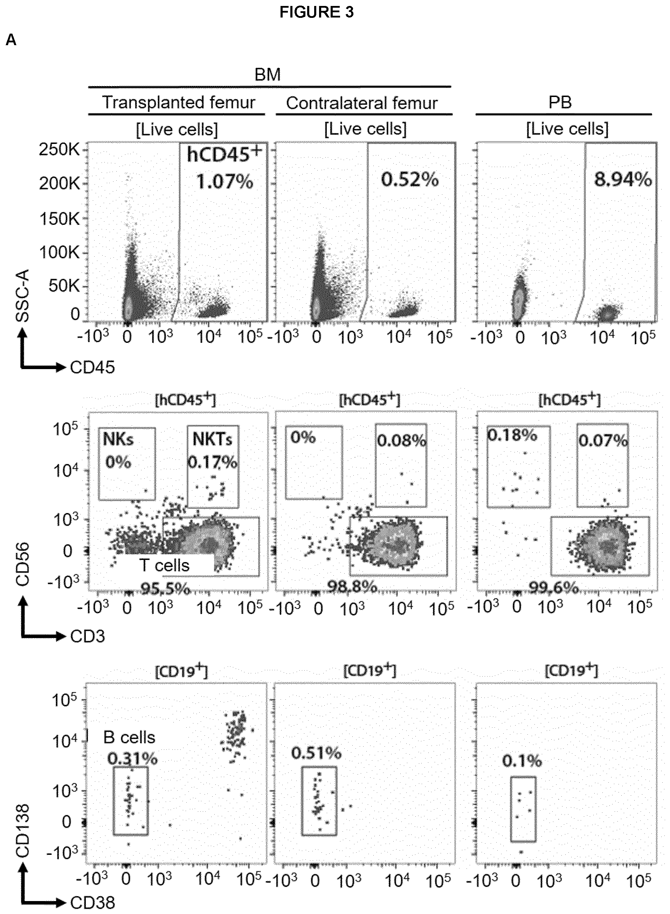

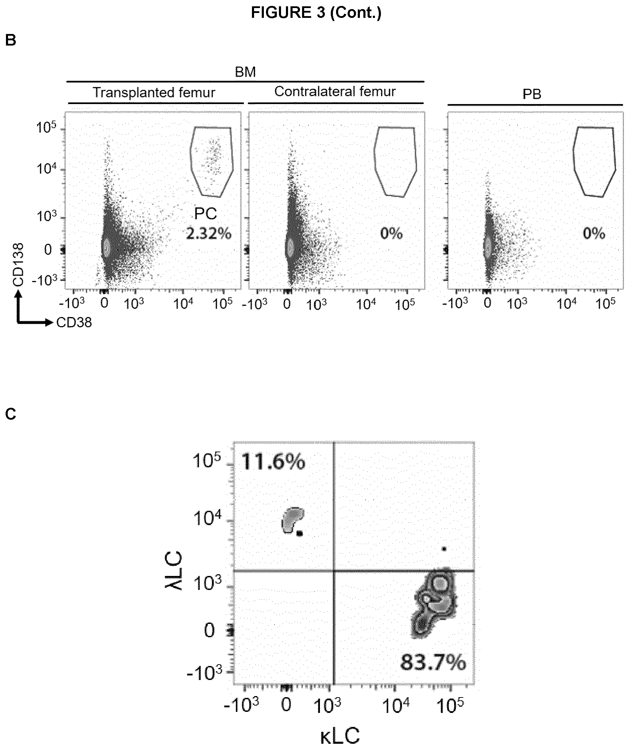

**Figure 3****.** Dot plots of a mouse transplanted with BMMCs from a relapsed patient sample with 0.99% CD34⁺ cells and 31.9% CD138⁺ CD38⁺ pathological plasma cells and kappa light chain isotype. A) Humanization in bone marrow (BM) of the transplanted femur, contralateral femur, and peripheral blood (PB). The percentages indicate the humanization and the ratio of NK cells, NKT cells, T lymphocytes, and B lymphocytes with respect to hCD45⁺ cells. **B**) Tumor engraftment of CD138⁺ CD38⁺ cells in the bone marrow (BM) of the transplanted femur, contralateral femur, and peripheral blood (PB). The percentages indicate the proportion of tumor engraftment with respect to the total human population. **C**) Intracellular staining of CD138⁺ CD38⁺ plasma cells for the determination of kappa (κLC) and lambda (λLC) light chains isotypes.

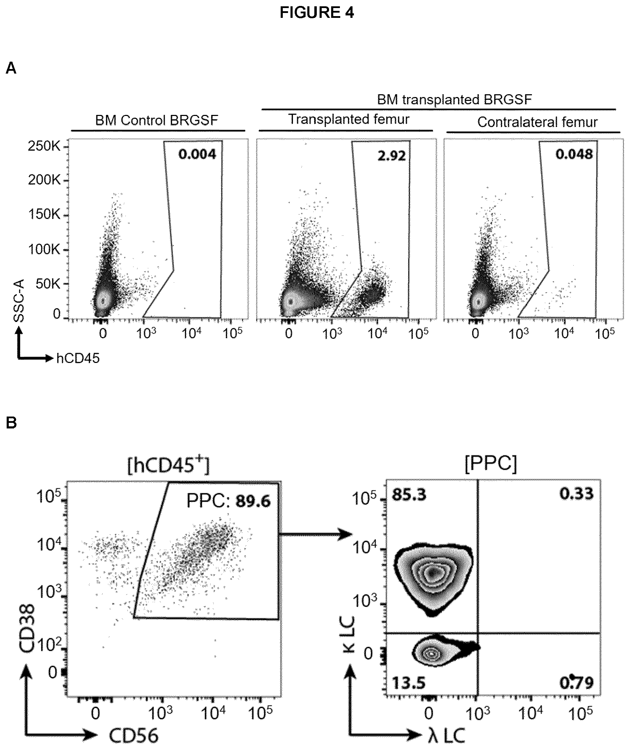

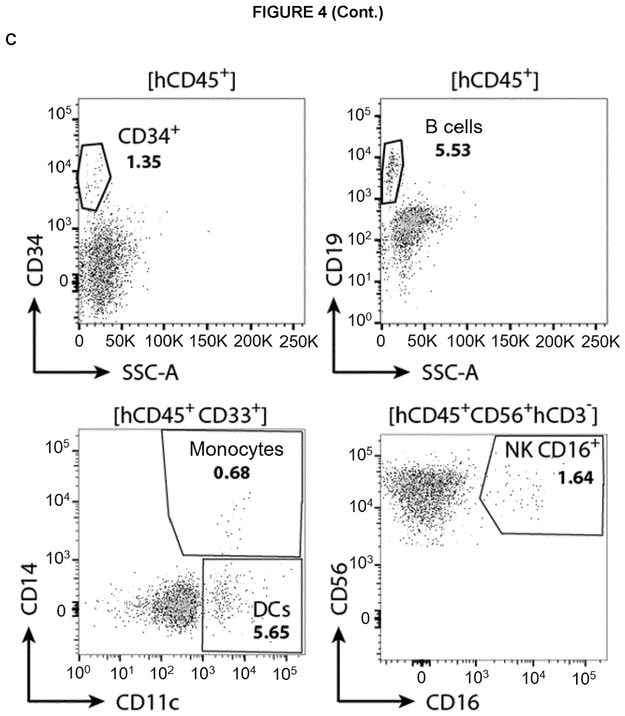

**Figure 4****.** Dot plots of a mouse transplanted with BMMCs from a relapsed patient sample with 1.47% CD34⁺ cells and 0.3% CD38⁺ CD56⁺ pathological plasma cells (PPC) and kappa light chain isotype. **A**) Humanization in bone marrow (BM) of the transplanted femur and contralateral femur, compared to the bone marrow of an untransplanted mouse. The percentages indicate humanization (percentage of human cells with respect to the total number of mouse cells). **B**) Tumor engraftment of CD38⁺ CD56⁺ cells in the bone marrow of the transplanted femur (percentage with respect to total human cells) and intracellular staining of plasma cells CD138⁺ CD38⁺ for the determination of kappa (κLC) and lambda (λLC) light chains isotypes. **C**) Percentages of the different healthy human immune populations detected (cDCs, monocytes, and CD34⁺ cells) in the bone marrow of the mouse, with respect to the total number of human cells.

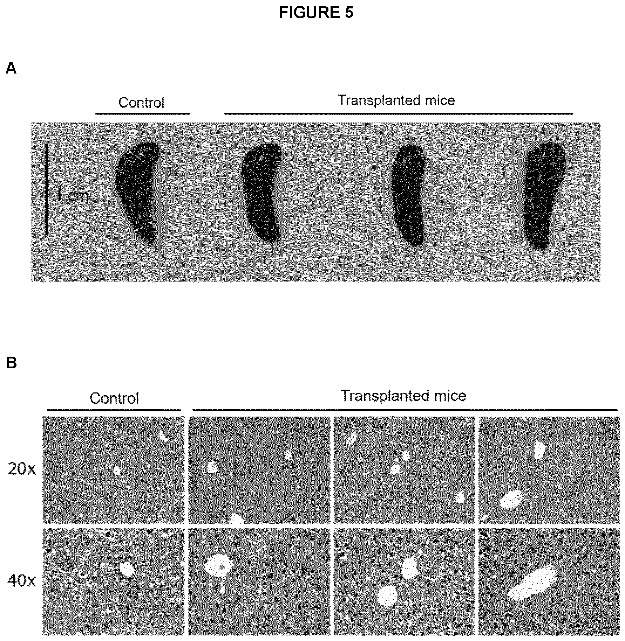

**Figure 5****.** Analysis of graft-versus-host-disease (Xeno-GvHD) in spleen and liver of transplanted mice. **A**) Representative photographs of spleens from a control mouse and three transplanted mice. **B**) Representative hematoxylin and eosin staining on histological samples of liver tissue from a control mouse and three transplanted mice.

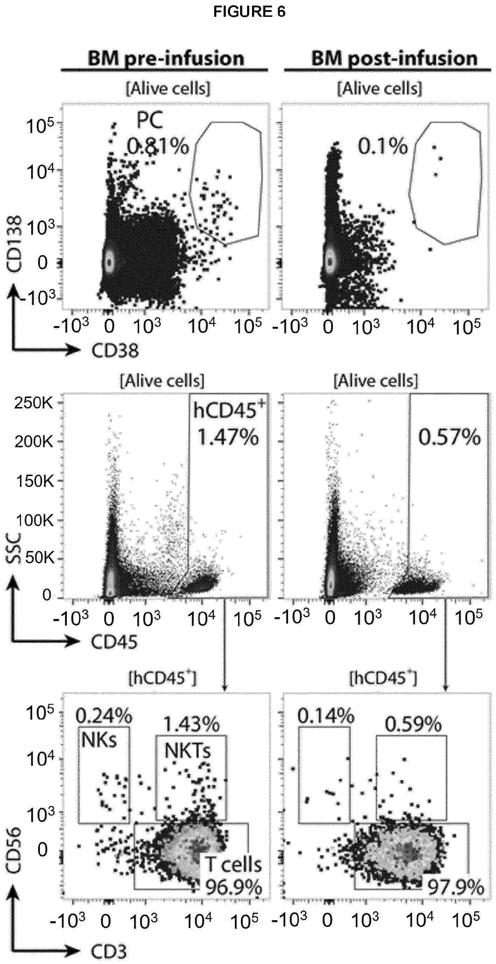

**Figure 6****.** Experimental study of the efficacy of CAR-NK immunotherapy. Six weeks after BMMCs injection, bone marrow aspirate was analyzed to confirm the existence of plasma cells. At week 8, 14×10⁶ BCMA-CAR-NK cells were intravenously injected and one week after treatment, the efficacy of the immunotherapy was tested. Flow cytometry dot plots before BCMA-CAR-NK cells infusion (left plots) and at necropsy after one week of treatment (right plots). Numbers indicate the percentage of tumor plasma cells within the total human compartment (top row) and healthy human immune engraftment (hCD45⁺ population; medium row). Bottom plots represent the percentage of the different healthy immune populations within the hCD45⁺ population. Arrows indicate the gating strategy.

### DESCRIPTION OF EMBODIMENTS

### Example 1. Production of the animal model for myeloma multiple

Female BRGSF mice (Genoway) were sublethally irradiated with a dose of 2 Gy and bone marrow (BM) mononuclear cells (BMMCs) from MM patient samples were transplanted by intra-femoral injection. The mice were humanized at 6-8 weeks of age. The graft reconstitution period lasted for 6 weeks. The animals received intraperitoneal potentiation with two agonist anti-human gp130 monoclonal antibodies B-P8 (#852080000, Diaclone) and B-S12 (#852110000, Diaclone) (whose mean half-life is 14.5 days) every fortnight during those 8 weeks, to guarantee the engraftment and the maintenance of the human tumor component in the mouse bone marrow. On the days indicated, the mice were infused with 50 µg of each anti-human gp130 monoclonal antibody. Both monoclonal antibodies specifically recognize the human GP130 protein but not the mouse one. After 6 weeks, peripheral blood (PB) and BM samples were extracted from the transplanted animals and the reconstitution of the main healthy human hematological lineages was analyzed by flow cytometry. From the same PB sample, the serum was extracted to analyze the presence of the patient's original human paraprotein. In those mice where the presence of humanization was confirmed, a new 2-week intraperitoneal injection of 5 µg hFLT3-L growth factor (BE0098, BioXCell) was carried out, to potentiate the accumulation of human myeloid cells. The mice were sacrificed, and the tumor and immune populations were analyzed by flow cytometry and histology (Figure 1).

6 BRGSF mice have been transplanted with BMMCs from MM patients (three relapses/progressions and three asymptomatic MM). Mice were sacrificed 8 weeks after transplantation. The humanization and graft success percentage obtained was 100%.

It was observed that the administration of anti-human gp130 monoclonal antibodies B-P8 and B-S12 and the growth factor hFLT3-L did not produce toxicity in the mice during the treatment.

The treatment of anti-human gp130 monoclonal antibodies B-P8 and B-S12 allowed engraftment and expansion of multiple myeloma for at least 8 weeks post-transplantation (19% average plasma graft in bone marrow) (Figure 2).

The percentages analyzed by flow cytometry of plasma cells (PC) and healthy immune populations in the bone marrow of the transplanted femur are shown in Table 1. The percentages are with respect to the total number of human cells in the mouse.

| **Table 1*** | |
|---|---|
| Cells | % cells |
| Plasma cells | 19.1 |
| CD34⁺ cells | 1.4 |
| T cells | 83.8 |
| B cells | 1.7 |
| NKT cells | 1.9 |
| NK cells | 3.2 |
| Dendritic cells | 3.2 |
| Monocytes | 1.3 |

| | |
|---|---|
| * Due to different and independent flow cytometry panels to identify each cell population, the sum of the percentages is not expected to be accurately 100%. | |

Table 2 compares the percentages of the immune populations present in the BM sample of the patients prior to the mice intra-femur infusion and in the BM of the transplanted mice at the time of necropsy. Values represent the mean of all transplanted mice (n = 6).

| **Table 2** | | |
|---|---|---|
| Cells | % cells in patient bone marrow sample | % cells in mouse bone marrow sample |
| Plasma cells | 7.1 | 19.1 |
| CD34⁺ cells | 1.4 | 1.3 |
| T cells | 11.9 | 83.8 |
| B cells | 2.1 | 1.7 |
| NKT cells | 2.5 | 1.9 |
| NK cells | 6.2 | 3.2 |
| Dendritic cells | 1.4 | 3.2 |
| Monocytes | 1.2 | 1.3 |

In mice transplanted with BMMCs from relapsed MM patients, the pathological plasma cells maintained the immunophenotype of the patient at the time of infusion (Figure 3B, Figure 4B). Furthermore, the plasma cells in mouse BM, at necropsy, maintained the dominant isotype of the original patient BM sample (Figure 3C, Figure 4B). In mice transplanted with BMMCs from patients with asymptomatic MM, expansion of other plasma populations that do not express the dominant light chain, was also observed.

In addition to the tumor graft, the mice presented healthy immune populations, preferably T lymphocytes (69.9% of the total human component in the bone marrow of the transplanted femur and 89.1% in peripheral blood) (Figure 2). The distribution of minority immune populations is variable among mice, but all the lineages of interest are represented: T lymphocytes, B lymphocytes, NK cells, NKT cells, CD34⁺ cells, and myeloid cells, such as CD14⁺ monocytes and dendritic cells (DC) (Figure 3A, Figure 4C). Furthermore, the proportions of these populations remained similar to those of the BM of origin, with the exception of T lymphocytes which could reflect a T cell expansion *in vivo* (Table 2).

Both the pathological plasma cells and the rest of the healthy populations remain preferentially in the BM of the transplanted femur. Pathological plasma cells have not been detected in peripheral blood. Healthy immune populations have been detected in peripheral blood (Figure 2, Figure 3A). Importantly, this situation reproduces what occurs in multiple myeloma patients, since the niche of multiple myeloma plasma cells is the bone marrow. Circulatory pathological cells are only detected in patients when the disease is localized extramedullary, in the context of a very aggressive gammopathy that can be secondary to multiple myeloma, known as plasma cell leukemia (plasma cell leukemia is characterized by having more than 20% of circulatory pathological cells). Another relevant aspect of the animal model is that no signs of graft-versus-host-disease (GvHD) have been detected, despite the presence of allogeneic T lymphocytes in the set of initially transplanted BMMCs and the large expansion of this cell type in the mouse. The size of the spleens at the time of sacrifice of the transplanted mice is similar to that of an untransplanted control mouse, indicating that there is no splenomegaly (Figure 5A). Likewise, liver histology samples showed normal tissue compared to that of the control, without apparent alterations characteristic of GvHD, such as lymphocyte infiltration, bile plugs (cholestasis), and tissue fibrosis (Figure 5B).

### Example 2. Use of the patient-derived humanized mouse model to predict the efficacy of a CAR-NK immunotherapy

The MM humanized mouse model of the invention is useful for testing the efficacy of treatments against patient-derived tumor plasma cells.

The MM humanized mouse model was generated as described in Example 1. A bone marrow sample was aspirated six weeks after BMMCs injection to analyze by flow cytometry the presence of tumor engraftment. Once plasma cells were detected in the transplanted bone marrow, at week 8, 14×10⁶ BCMA-CAR-NK cells were intravenously injected and one week after the animal was sacrificed, and efficacy of the therapy was measured.

One single BCMA-CAR-NK cells infusion was able to achieve an 8-fold reduction in tumor burden (from 0.8% of plasma cells within the total human component to 0.1%) proving the efficacy of the therapy (Figure 6, top dot plots). Human healthy engraftment (hCD45⁺ population) was also reduced but in a lower fold (2.6-fold reduction; Figure 6, middle dot plots) and the different immune populations remained in the same proportion, which shows the specificity of the CAR-NK immunotherapy (Figure 6, bottom dot plots).

These results show that the patient-derived humanized mouse model is useful for studying the efficacy of therapies against multiple myeloma in a near-immunocompetent environment in which immune healthy components such as NK, NKT, T, and B cells from the same patient are also present. These results were obtained with just one mouse in a sequential model (1 mouse/1 patient/1 CAR immunotherapy). This sequential model with just one mouse represents a proof of concept which allows reducing the number of mice necessary to predict the efficacy of an immunotherapy in a patient.

### CITATION LIST

Calimeri et al. (2011). A unique three-dimensional SCID-polymeric scaffold (SCID-synth-hu) model for in vivo expansion of human primary multiple myeloma cells. Leukemia, 25(4), 707-11. doi: 10.1038/leu.2010.300
Das et al. (2016). Microenvironment-dependent growth of preneoplastic and malignant plasma cells in humanized mice. Nat Med, 22(11), 1351-1357. doi:10.1038/nm.4202
Libouban (2015). The use of animal models in multiple myeloma. Morphologie, 99(325), 63-72. doi:10.1016/j.morpho.2015.01.003
Lopez-Lastra et al. (2017). A functional DC cross talk promotes human ILC homeostasis in humanized mice. Blood Adv., 1(10), 601-614. doi:10.1182/bloodadvances.2017004358
Rebouissou et al. (1998). A gp130 interleukin-6 transducer-dependent SCID model of human multiple myeloma. Blood, 91(12), 4727-37.
Rossi et al. (2018). Mouse models of multiple myeloma: technologic platforms and perspectives. Oncotarget, 9(28), 20119-20133. doi:10.18632/oncotarget.24614
Yaccoby et al. (1998). Primary myeloma cells growing in SCID-hu mice: a model for studying the biology and treatment of myeloma and its manifestations. Blood, 92(8), 2908-13.

## Claims

1. A human immune system transgenic mouse having the genotype Rag2^{-/-}, Il2rg^{-/-}, Sirpa^{NOD}, Flt3^{-/-},
(a) engrafted with bone marrow mononuclear cells (BMMCs) obtained from a subject having multiple myeloma;
(b) administered with antibodies recognizing human gp130; and
(c) administered with human FLT3 ligand (hFLT3-L).

2. The mouse according to claim 1, wherein the mouse has been irradiated prior to engraftment with BMMCs.

3. The mouse according to claim 1 or 2, wherein the BMMCs cells are engrafted intrafemorally.

4. The mouse according to any one of claims 1 to 3, wherein the administration of the antibodies recognizing human gp130 is repeated every 2 weeks, until week 8 from engraftment.

5. The mouse according to any one of claims 1 to 4, wherein the agonist antibodies recognizing human gp130 are administered intraperitoneally.

6. The mouse according to any one of claims 1 to 5, wherein hFLT3-L is administered 3 times per week, for 2 weeks, starting at week 6 from engraftment.

7. The mouse according to any one of claims 1 to 6, wherein the agonist antibodies recognizing human gp130 are the antibodies B-P8 and B-S12.

8. The mouse according to any one of claims 1 to 7, wherein hFLT3-L is administered intraperitoneally.

9. A method for predicting the anti-tumour response of a subject to a multiple myeloma treatment, comprising:
(a) preparing a human immune system transgenic mouse of any one of claims 1 to 8 by:
(i) engrafting the mouse with bone marrow mononuclear cells (BMMCs) obtained from a subject having multiple myeloma;
(ii) administering antibodies recognizing human gp130 to the mouse; and
(iii) administering human FLT3 ligand (hFLT3-L) to the mouse;
(b) administering a multiple myeloma treatment to the mouse;
(c) evaluating the anti-tumour response of the mouse to the multiple myeloma treatment; and
(d) predicting the anti-tumour response of the subject to the multiple myeloma treatment based on the evaluation performed in step (c).

10. The method according to claim 9, wherein the multiple myeloma treatment is selected from the group consisting of radiation therapy, gene therapy, and adoptive cell therapy.

11. The method according to claim 10, wherein the adoptive cell therapy uses cells selected from the group consisting of Natural Killer (NK) cells, T cells, macrophages, Natural Killer T (NKT) cells, gamma delta T (γδ T) cells, CAR-NK cells, CAR-T cells, CAR-macrophage cells, CAR-NKT cells, and CAR-γδ T cells.

12. The method according to claim 9, wherein the multiple myeloma treatment comprises the administration of a chemotherapeutic agent or an antibody.

13. The method according to claim 9, wherein the multiple myeloma treatment is administered intravenously.
